Europäisches Patentamt

European Patent Office

Office européen des brevets

(11) Veröffentlichungsnummer: **0 256 458 B1**

(12) **EUROPÄISCHE PATENTSCHRIFT**

(45) Veröffentlichungstag der Patentschrift: **29.04.92**

(21) Anmeldenummer: **87111491.4**

(22) Anmeldetag: **08.08.87**

(51) Int. Cl.5: **A61K 7/11, C08F 226/10,**
**C08F 220/54, C08F 220/10**

(54) **Verfahren zur Herstellung eines Copolymerisates zur Verwendung als Haarfixiermittel und dieses enthaltende Haarfixiermittel.**

(30) Priorität: **18.08.86 DE 3627969**

(43) Veröffentlichungstag der Anmeldung:
**24.02.88 Patentblatt 88/08**

(45) Bekanntmachung des Hinweises auf die
Patenterteilung:
**29.04.92 Patentblatt 92/18**

(84) Benannte Vertragsstaaten:
**DE ES FR GB IT NL**

(56) Entgegenhaltungen:
**EP-A- 0 062 002**
**DE-A- 2 047 655**
**DE-A- 2 715 297**

(73) Patentinhaber: **BASF Aktiengesellschaft**
**Carl-Bosch-Strasse 38**
**W-6700 Ludwigshafen(DE)**

(72) Erfinder: **Nuber, Adolf, Dr.**
**Kurt-Schumacher-Strasse 11**
**W-6737 Boehl-Iggelheim(DE)**
Erfinder: **Sanner, Axel, Dr.**
**Lorscher Ring 2 c**
**W-6710 Frankenthal(DE)**
Erfinder: **Vogel, Friedrich, Dr.**
**Im kleinen Letten 3**
**W-6706 Wachenheim(DE)**
Erfinder: **Mass, Dietrich**
**Nibelungenstrasse 13**
**W-6704 Mutterstadt(DE)**

**Beschreibung**

Die vorliegende Erfindung betrifft ein Verfahren zur Herstellung eines Copolymerisates zur Verwendung als Haarfixiermittel und dieses enthaltende Haarfixiermittel.

Für die Haarkosmetik werden die verschiedensten Polymeren eingesetzt. Die bisherigen Copolymere sind häufig unzureichend verträglich mit den in Haarsprays verwendeten unpolaren Treibgasen auf Alkanbasis, die aus ökologischen Gründen in zunehmendem Maße als Ersatz für Fluorkohlenwasserstoffe Verwendung finden. Auch läßt in manchen Fällen die Haarfestigerwirkung zu wünschen übrig.

In der EP-B1-37 378 wird ein Harz beschrieben das die genannten Forderungen weitgehend erfüllt, dessen außerordentlich komplizierte und aufwendige Herstellung in Form einer genau kontrollierten Lösungspolymerisation nachteilig ist.

Das Ziel der vorliegenden Erfindung besteht in der Bereitstellung eines möglichst einfach herstellbaren Copolymeren mit besonders guten haarfestigenden Eigenschaften und guter Verträglichkeit, d.h. Löslichkeit, mit den halogenfreien, unpolaren Treibgasen Propan und Butan sowie guter Löslichkeit in den üblicherweise verwendeten polaren Lösungsmitteln, insbesondere Alkoholen.

Demgemäß wurde ein Verfahren zur Herstellung eines Copolymerisates zur Verwendung als Haarfixiermittel gefunden, welches dadurch gekennzeichnet ist, daß man

A. 20 bis 60 Gew.-% Vinylpyrrolidon,

B. 20 bis 60 Gew.-% eines am N-Atom mono- oder dialkylierten Acrylamids mit 1 bis 8 C-Atomen im Alkyl oder ihren Mischungen und

C. 5 bis 60 Gew.-% eines Alkyl- oder Hydroxyalkylesters der Acryl-oder Methacrylsäure mit 1 bis 4 C-Atomen im Alkyl oder 2 bis 4 C-Atomen im Hydroxyalklrest oder Mischungen dieser Ester oder

3 bis 12 Gew.-% Acrylsäure oder Methacrylsäure oder

2 bis 48 Gew.-% eines Alkyl- oder Hydroxyalkylesters der Acrylsäure oder Methacrylsäure mit 1 bis 4 C-Atomen im Alkyl oder 2 bis 4 C-Atomen im Hydroxyalkylrest oder Mischungen dieser Ester und

3 bis 12 Gew.-% Acrylsäure oder Methacrylsäure,

wobei die Gew.-% bezogen sind auf das Gesamtgewicht der Monomeren,

radikalisch miteinander polymerisiert, indem man die Monomeren langsam zu einem polymerisierenden Gemisch in einem Lösungsmittel zudosiert, wobei das erhaltene Copolymerisat in niedrigen Alkoholen mit 1 bis 4 C-Atomen löslich ist und einen K-Wert von 15 bis 75 aufweist.

In einer bevorzugten Ausführungsform stellt man ein solches Copolymerisat dadurch her, daß man

A. 30 bis 50 Gew.-% Vinylpyrrolidon,

B. 30 bis 50 Gew.-% eines am N-Atom mono- oder dialkylierten Acrylamids mit 1 bis 8 C-Atomen im Alkyl und

C. 20 bis 40 Gew.-% eines Alkyl- oder Hydroxyalkylesters der Acryl-oder Methacrylsäure mit 1 bis 4 C-Atomen im Alkyl oder 2 bis 4 C-Atomen im Hydroxyalkylrest oder Mischungen dieser Ester oder

5 bis 10 Gew.-% Acrylsäure oder Methacrylsäure oder

15 bis 35 Gew.-% eines Alkyl- oder Hydroxyalkylesters der Acrylsäure oder Methacrylsäure mit 1 bis 4 C-Atomen im Alkyl oder 2 bis 4 C-Atomen im Hydroxyalkylrest oder Mischungen dieser Ester und

5 bis 10 Gew.-% Acrylsäure oder Methacrylsäure,

radikalisch miteinander polymerisiert, wobei das erhaltene Copolymerisat einen K-Wert von 20 bis 50 aufweist.

Gegenstand der Erfindung ist auch ein Haarfixiermittel, enthaltend als Filmbildner das oben bezeichnete Copolymerisat, dessen gegebenenfalls vorhandene Carboxylgruppen in neutralisierter oder teilneutralisierter Form vorliegen können, und dessen Verwendung als Filmbildner in Haarfixiermitteln.

N-Mono- oder N,N-Dialkylacrylamide sind beispielsweise:

N,N-Dimethylacrylamid,

N,N-Diethylacrylamid,

N,N-Dibutylacrylamid,

N,N-Dipentylacrylamid und

N-tert.-Butyl-, N-sek.-Butyl-, N-n-Butyl-, N-n-Octyl- oder

N-tert.-Octylacrylamid.

Davon sind bevorzugt N-tert.-Butyl-, N-n-Octyl- und N,N-Dimethylacrylamid sowie Mischungen dieser Monomeren.

Acryl- oder Methacrylester sind beispielsweise Methacrylat, Methylmethacrylat, Ethylacrylat, Ethylmethacrylat, tert.-Butylacrylat, tert.-Butylmethacrylat, Hydroxypropylacrylat in Form seines technischen Gemisches aus 2- und 3-Hydroxypropylacrylat.

EP 0 256 458 B1

Falls Acrylsäure oder Methacrylsäure als Monomere eingesetzt werden, dienen diese carboxylgruppentragenden Monomeren zur Verbesserung der Löslichkeit in Wasser, wobei die Carboxylgruppen teilweise, d.h. zu mindestens 50 %, oder vollständig, d.h. zu 100 %, durch ein organisches Amin, vorzugsweise ein Mono-, Di- oder Trialkanolamin mit insgesamt 2 bis 9 C-Atomen, wie 2-Amino-2-methylpropanol, 2-Amino-2-methyl-1,3-propandiol, Triethanolamin oder Triisopropanolamin, neutralisiert sind.

In den Fällen, in denen Acrylester als Monomere verwendet werden, die eine Löslichkeit in Wasser oder wäßrigen Lösungen herabsetzen können, liegt in der Regel der Gehalt an Vinylpyrrolidon, das die Löslichkeit erhöht, bei wenigstens 30 %.

Das erfindungsgemäß hergestellte Copolymerisat weist den oben genannten definierten K-Wert auf und muß in einem niederen Alkohol mit 1 bis 4 C-Atomen, insbesondere in Ethanol und/oder Isopropanol, löslich sein. Bei einpolymerisierten Acryl- und/oder Methacrylsäuregruppen soll es sowohl in der Säureform als auch in der oben definierten neutralisierten oder teilneutralisierten Form in einem niederen Alkohol löslich sein.

Die bezeichneten Copolymeren können nach den üblichen Polymerisationsverfahren der Lösungs-, Fällungs- oder Suspensionspolymerisation hergestellt werden. Notwendig ist ein Verfahren, bei dem die Monomeren langsam zu einem polymerisierenden Gemisch zudosiert werden, da so ein zweckmäßiger Verlauf der Polymerisation gewährleistet wird. Als Initiatoren für die Polymerisation werden die üblichen Peroxide, wie Benzoylperoxid, tert.-Butylperpivalat, tert.-Butylper-2-ethylhexanoat, Di-tert.-butylperoxid, tert.-Butylhydroperoxid u.a. sowie Azostarter, wie Azo-bis-isobutyronitril, in Mengen von 0,5 bis 3 Gew.%, bezogen auf das Gewicht der Monomeren, verwendet.

Die K-Werte werden gemäß Fikentscher, Cellulosechemie, 13, 58-64 (1932) bei 25°C in Ethanol als Lösungsmittel in 1 gew.%iger Lösung, bei den carboxylgruppenhaltigen Copolymerisaten in der Säureform, bestimmt.

Darüber hinaus kann man die erfindungsgemäßen Copolymerisate durch ihre Glastemperatur (Glasübergangstemperatur), die wenigstens ca. 60°C betragen soll, charakterisieren. Der bevorzugte Bereich liegt bei 80 bis 1700°C.

Die bezeichneten Copolymerisate werden vorteilhaft als Filmbildner in Haarfixiermittel in den üblichen Zubereitungen und Mitteln, wobei vorhandene Carboxylgruppen zweckmäßig zu 50 bis 100 %, neutralisiert sind, in an sich üblicher Weise mit gegebenenfalls zusätzlichen, üblicherweise verwendeten Additiven, wie Riechstoffen, Weichmachern, Farbstoffen usw. eingesetzt. Bevorzugte Zubereitungen sind Sprays und Schäume.

Besonders bevorzugte Haarfixierzubereitungen sind Haarsprays mit den bevorzugten Lösungsmitteln Ethanol und/oder Isopropanol, gegebenenfalls in Kombination mit 1,0 bis 15,0 Gew.% Wasser oder 1,0 bis 40,0 Gew.% eines anderen Lösungsmittels, wie Methylenchlorid oder 1,1,1-Trichlorethan.

Bevorzugte Kombinationen bestehen aus 1,0 bis 5,0, bevorzugt 1,5 bis 3 Gew.%, neutralisiertem oder teilneutralisiertem Copolymerisat, 50 bis 75 Gew.% Lösungsmittel, ausgewählt aus der Gruppe, bestehend aus einem niederen Alkohol mit 2 und 3 C-Atomen, Methylenchlorid oder 1,1,1-Trichlorethan und ihren Mischungen und 25 bis 50 Gew.% Propan und/oder Butan als Treibgas. Gegebenenfalls kann in geringer Menge Trichlorfluormethan als Treibgas mitverwendet werden, etwa in Mengen bis zu 20 Gew.%, bezogen auf Treibgas.

Die erfindungsgemäßen Haarfixierzubereitungen enthalten in der Regel, bezogen auf das Gesamtgewicht 0,5 bis 5, bevorzugt 1,5 bis 3 Gew.% Copolymerisat.

Beispiele für die Herstellung

Beispiel 1

In einen 1-Liter-Kolben mit Rührer, Rückflußkühler und zwei Zulauftropftrichtern werden 10 % einer Mischung aus 180 Teilen Vinylpyrrolidon, 80 Teilen N-tert.-Butylacrylamid, 40 Teilen Ethylacrylat und 300 Teilen Isopropanol sowie 10 % einer Mischung aus 50 Teilen Isopropanol und 4,5 Teilen tert.-Butylperpivalat vorgelegt, der Inhalt bis zum Sieden hochgeheizt und unter Rühren die restlichen 90 % der Mischungen innerhalb von 8 Stunden gleichmäßig zugefahren. Es entsteht eine klare, gelbliche, viskose Lösung. Nach Beendigung des Zufahrens wird noch zwei Stunden bei der Siedetemperatur belassen und dann abgekühlt.

Nach den Angaben gemäß Beispiel 1 wurden folgende Polymere hergestellt.

Tabelle

| Beispiel | Gewichtsteile | | | | | | | | |
|---|---|---|---|---|---|---|---|---|---|
| | 1 | 2 | 3 | 4 | 5 | 6 | 7 | 8 | |
| Vinylpyrrolidon | 180 | 90 | 120 | 120 | 150 | 100 | 75 | 90 | 85 |
| N,N-Dimethylacrylamid | - | - | - | 120 | - | 90 | 75 | 90 | 180 |
| N-n-Butylacrylamid | - | - | - | - | 90 | - | 150 | - | - |
| N-tert.-Butylacrylamid | 80 | 120 | - | - | - | 100 | - | - | - |
| N-n-Octylacrylamid | - | - | 120 | - | - | - | - | - | - |
| Methylacrylat | - | - | 30 | - | - | - | - | 120 | - |
| Methylmethacrylat | - | - | - | - | 60 | - | - | - | - |
| Ethylacrylat | 40 | - | - | - | - | - | - | - | - |
| n-Butylacrylat | - | 60 | - | - | - | - | - | - | - |
| tert.-Butylacrylat | - | - | - | 60 | - | - | - | - | - |
| Acrylsäure | - | 30 | - | - | - | 10 | - | - | 35 |
| Methacrylsäure | - | - | 30 | - | - | - | - | - | - |

Beispiele für Zubereitungen

Haarsprayformulierungen

| 1. | Copolymerisat gemäß Beispiel 1 | 2,0 Gew.% |
|---|---|---|
| | Ethanol/Isopropanol 50:50 | 68,0 Gew.% |
| | Propan/Butan 40 : 60 | 30,0 Gew.% |
| 2. | Copolymerisat gemäß Beispiel 1 | 3,0 Gew.% |
| | Ethanol | 57,0 Gew.% |
| | Methylenchlorid | 10,0 Gew.% |
| | Propan/Butan 40 : 60 | 30,0 Gew.% |
| 3. | Copolymerisat gemäß Beispiel 1 | 2,0 Gew.% |
| | Ethanol | 33,0 Gew.% |
| | Methylenchlorid | 35,0 Gew.% |
| | Propan/Butan 40 : 60 | 30,0 Gew.% |

**Patentansprüche**

1. Verfahren zur Herstellung eines Copolymerisats zur Verwendung als Haarfixiermittel, dadurch gekennzeichnet, daß man

A. 20 bis 60 Gew.-% Vinylpyrrolidon,

B. 20 bis 60 Gew.-% eines am N-Atom mono- oder dialkylierten Acrylamids mit 1 bis 8 C-Atomen im Alkyl oder ihren Mischungen und

C. 5 bis 60 Gew.-% eines Alkyl- oder Hydroxyalkylesters der Acryl-oder Methacrylsäure mit 1 bis 4 C-Atomen im Alkyl oder 2 bis 4 C-Atomen im Hydroxyalkylrest oder Mischungen dieser Ester oder

3 bis 12 Gew.-% Acrylsäure oder Methacrylsäure oder

2 bis 48 Gew.-% eines Alkyl- oder Hydroxyalkylesters der Acrylsäure oder Methacrylsäure mit 1 bis 4 C-Atomen im Alkyl oder 2 bis 4 C-Atomen im Hydroxyalkylrest oder Mischungen dieser Ester und

3 bis 12 Gew.-% Acrylsäure oder Methacrylsäure,

wobei die Gew.-% bezogen sind auf das Gesamtgewicht der Monomeren,

radikalisch miteinander polymerisiert, indem man die Monomeren langsam zu einem polymerisierenden Gemisch in einem Lösungsmittel zudosiert, wobei das erhaltene Copolymerisat in niedrigen Alkoholen mit 1 bis 4 C-Atomen löslich ist und einen K-Wert von 15 bis 75 aufweist.

2. Verfahren nach Anspruch 1, dadurch gekennzeichnet, daß man

A. 30 bis 50 Gew.-% Vinylpyrrolidon,

B. 30 bis 50 Gew.-% eines am N-Atom mono- oder dialkylierten Acrylamids mit 1 bis 8 C-Atomen im Alkyl und

C. 20 bis 40 Gew.-% eines Alkyl- oder Hydroxyalkylesters der Acryl-oder Methacrylsäure mit 1 bis 4 C-Atomen im Alkyl oder 2 bis 4 C-Atomen im Hydroxyalkylrest oder Mischungen dieser Ester oder

5 bis 10 Gew.-% Acrylsäure oder Methacrylsäure oder

15 bis 35 Gew.-% eines Alkyl- oder Hydroxyalkylesters der Acrylsäure oder Methacrylsäure mit 1 bis 4 C-Atomen im Alkyl oder 2 bis 4 C-Atomen im Hydroxyalkylrest oder Mischungen dieser Ester und

5 bis 10 Gew.-% Acrylsäure oder Methacrylsäure,

radikalisch miteinander polymerisiert, wobei das erhaltene Copolymerisat einen K-Wert von 20 bis 50 aufweist.

3. Verfahren nach Anspruch 1 oder 2, dadurch gekennzeichnet, daß man die freien Carboxylgruppen des erhaltenen Copolymerisats zu 50 bis 100 % durch ein Mono-, Di- oder Trialkanolamin mit insgesamt 2 bis 9 C-Atomen neutralisiert.

4. Haarfixiermittel, enthaltend als Filmbildner ein gemäß Anspruch 1 bis 3 hergestelltes Copolymerisat.

5. Haarfixiermittel nach Anspruch 4, enthaltend den Filmbildner in einer Menge von 0,5 bis 5 Gew.-%, bezogen auf das Gesamtgewicht.

6. Haarsprayzubereitungen nach Anspruch 4, enthaltend, bezogen auf das Gesamtgewicht, 1,0 bis 5,0 Gew.-% eines gemäß Anspruch 3 hergestellten Copolymerisats, 50 bis 75 Gew.-% Lösungsmittel und 25 bis 50 Gew.-% Treibgas.

7. Verwendung von gemäß Anspruch 1 bis 3 hergestellten Copolymerisaten als Filmbildner in Haarfixiermitteln.

**Claims**

1. A process for preparing a copolymer for use as a hair setting agent, which comprises subjecting

A. from 20 to 60% by weight of vinylpyrrolidone,

B. from 20 to 60% by weight of an acrylamide which is monoalkylated or dialkylated at the N atom, with 1 to 8 carbon atoms in the alkyl, or mixtures thereof and

C. from 5 to 60% by weight of an alkyl or hydroxyalkyl ester of acrylic or methacrylic acid having 1 to 4 carbon atoms in the alkyl or 2 to 4 carbon atoms in the hydroxyalkyl or mixtures thereof or

from 3 to 12% by weight of acrylic acid or methacrylic acid or

from 2 to 48% by weight of an alkyl or hydroxyalkyl ester of acrylic acid or methacrylic acid having 1 to 4 carbon atoms in the alkyl or 2 to 4 carbon atoms in the hydroxyalkyl or mixtures thereof and

from 3 to 12% by weight of acrylic acid or methacrylic acid,

the % by weight being based on the total weight of the monomers, to free-radical polymerization by slowly adding the monomers to a polymerizing mixture in a solvent, the copolymer obtained being soluble in lower alcohols of 1 to 4 carbon atoms and having a K value of from 15 to 75.

2. A process as claimed in claim 1, wherein

A. from 30 to 50% by weight of vinylpyrrolidone,

B. from 30 to 50% by weight of an acrylamide which is monoalkylated or dialkylated at the N atom, with 1 to 8 carbon atoms in the alkyl and

C. from 20 to 40% by weight of an alkyl or hydroxyalkyl ester of acrylic or methacrylic acid having 1 to 4 carbon atoms in the alkyl or 2 to 4 carbon atoms in the hydroxyalkyl or mixtures thereof or

from 5 to 10% by weight of acrylic acid or methacrylic acid or

from 15 to 35% by weight of an alkyl or hydroxyalkyl ester of acrylic acid or methacrylic acid having 1 to 4 carbon atoms in the alkyl or 2 to 4 carbon atoms in the hydroxyalkyl or mixtures thereof and

from 5 to 10% by weight of acrylic acid or methacrylic acid,

are subjected to free-radical polymerization, the copolymer obtained having a K value of from 20 to 50.

**3.** A process as claimed in claim 1 or 2, wherein the free carboxyl groups of the copolymer obtained are neutralized to from 50 to 100% with a mono-, di- or trialkanolamine having a total of 2 to 9 carbon atoms.

**4.** A hair setting composition containing a copolymer prepared as claimed in claim 1 or 2 or 3 as a film former.

**5.** A hair setting composition as claimed in claim 4, containing the film former in an amount of from 0.5 to 5% by weight, based on the total weight.

**6.** A hair spray preparation as claimed in claim 4, containing, based on the total weight, from 1.0 to 5.0% by weight of a copolymer prepared as claimed in claim 3, from 50% to 75% by weight of solvent and from 25 to 50% by weight of propellant.

**7.** Use of a copolymer prepared as claimed in claim 1 or 2 or 3 as a film former in hair setting compositions.

**Revendications**

**1.** Procédé de préparation d'un copolymère destiné à être utilisé comme fixateur pour cheveux, caractérisé en ce qu'on polymérise conjointement, par polymérisation radicalaire,
A. de 20 à 60% en poids de vinylpyrrolidone,
B. de 20 à 60% en poids d'un acrylamide mono- ou dialkylé sur l'atome d'azote, renfermant 1 à 8 atomes de carbone dans le reste alkyle, ou de mélanges de ces acrylamides, et
C. de 5 à 60% en poids d'un ester alkylique ou hydroxyalkylique de l'acide acrylique ou méthacrylique, renfermant 1 à 4 atomes de carbone dans le reste alkyle ou 2 à 4 atomes de carbone dans le reste hydroxyalkyle, ou de mélanges de ces esters, ou
de 3 à 12% en poids d'acide acrylique ou d'acide méthacrylique, ou
de 2 à 48% en poids d'un ester alkylique ou hydroxyalkylique de l'acide acrylique ou de l'acide méthacrylique renfermant 1 à 4 atomes de carbone dans le reste alkyle ou 2 à 4 atomes de carbone dans le reste hydroxyalkyle, ou de mélanges de ces esters et
de 3 à 12% en poids d'acide acrylique ou d'acide méthacrylique,
les pourcentages en poids se rapportant au poids total des monomères
en ajoutant les monomères lentement jusqu'à un mélange polymérisant dans un solvant, le copolymère obtenu étant soluble dans les alcools inférieurs à 1-4 atomes de carbone et présentant une valeur K de 15 à 75.

**2.** Procédé selon la revendication 1, caractérisé en ce qu on polymérise conjointement, par polymérisation radicalaire,
A. de 30 à 50% en poids de vinylpyrrolidone,
B. de 30 à 50% en poids d'un acrylamide mono- ou dialkylé sur l'atome d'azote, renfermant 1 à 8 atomes de carbone dans le reste alkyle, et
C. de 20 à 40% en poids d'un ester alkylique ou hydroxyalkylique de l'acide acrylique ou méthacrylique, renfermant 1 à 4 atomes de carbone dans le reste alkyle ou 2 à 4 atomes de carbone dans le reste hydroxyalkyle, ou de mélanges de ces esters, ou
de 5 à 10% en poids d'acide acrylique ou d'acide méthacrylique, ou
de 15 à 35% en poids d'un ester alkylique ou hydroxyalkylique de l'acide acrylique ou de l'acide méthacrylique renfermant 1 à 4 atomes de carbone dans le reste alkyle ou 2 à 4 atomes de carbone dans le reste hydroxyalkyle, ou de mélanges de ces esters et
de 5 à 10% en poids d'acide acrylique ou d'acide méthacrylique,
le copolymère obtenu présentant une valeur K de 20 à 50.

**3.** Procédé selon la revendication 1 ou 2, caractérisé en ce qu'on neutralise les groupements carboxyle libres du copolymère obtenu, à raison de 50 à 100%, par une mono-, di- ou trialcanolamine renfermant au total 2 à 9 atomes de carbone.

**4.** Fixateur pour cheveux contenant, en tant que filmogène, un copolymère selon l'une quelconque des revendications 1 à 3.

**5.** Fixateur pour cheveux selon la revendication 4, contenant le filmogène dans une proportion de 0,5 à 5% en poids par rapport au poids total.

**6.** Préparations de laque capillaire pulvérisable selon la revendication 4, contenant, par rapport au poids total, de 1,0 à 5,0% en poids d'un copolymère préparé selon la revendication 3, de 50 à 75% en poids de solvant et de 25 à 50% en poids de gaz propulseur.

**7.** Utilisation de copolymères préparés selon l'une quelconque des revendications 1 à 3, comme filmogène dans des fixateurs pour cheveux.